# EUROPÄISCHE PATENTSCHRIFT

(11) **EP 0 366 018 B1**
(45) Veröffentlichungstag und Bekanntmachung des Hinweises auf die Patenterteilung: **26.05.1993**
(21) Anmeldenummer: 89119517.4
(22) Anmeldetag: 20.10.1989
(51) Int. Cl.: A61L 27/00, A61F 2/28

(54) **Metallspongiosa und Verfahren zu ihrer Herstellung**
Metal sponge-like structure and method for making the same
Structure cellulaire métallique et son procédé de fabrication

(30) Priorität: 24.10.1988 DD 320999; 24.10.1988 DD 321000
(43) Veröffentlichungstag der Anmeldung: 02.05.1990
(73) Patentinhaber: Krysmann, Waldemar, Dr.rer.nat., D-09130 Chemnitz (DE); Morgenstern, Rainer, Dr.med., D-09112 Chemnitz (DE); Rabending, Klaus, Dr.rer.nat., 09249 Taura (DE)
(72) Erfinder: Kurze, Peter, Prof. Dr. Sc. nat., DD-9109 Oberlichtenau (DD); Rabending, Klaus, Dr. rer. nat., DD-9115 Taura (DD); Morgenstern, Rainer, Dr. med., DD-9005 Karl-Max-Stadt (DD); Krysmann, Waldemar, Dr. rer. nat., DD-9071 Karl-Max-Stadt (DD); Daniel, Peter, Doz. Dr. sc. med., DD-9081 Karl-Max-Stadt (DD); Polster, Manfred, DD-9030 Karl-Max-Stadt (DD)
(74) Vertreter: Hansen, Bernd, Dr. Dipl.-Chem.

(56) Entgegenhaltungen:
- EP-A- 0 237 053
- DE-A- 2 910 627
- GB-A- 2 093 701

## Beschreibung

Die Erfindung betrifft eine Metallspongiosa aus Titanium oder Tantal oder deren Legierungen und ein Verfahren zu ihrer Herstellung. Die Metallspongiosaherstellung findet in der Medizintechnik Anwendung.
Die Metallspongiosa werden als Knochenersatz in der Humanmedizin verwendet.

Bekannt sind metallische Dauerimplantate aus bioinerten reinen Metallen bzw. deren Legierungen, die beispielsweise in Form von Gelenkprothesen in der Chirurgie Anwendung finden und deren fester Kontakt mit dem Knochen mit Hilfe von Knochenzement erfolgt.

Wegen der allmählichen Strukturveränderungen des Knochenzementes kommt es zur Auslockerung und zur Liegezeitverkürzung des Implantates. Aus diesem Grund werden zementfreie Implantat-Knochenverbindungen gewünscht. Bekannt hierzu sind die Oberflächen der Metallimplantate in geeigneter Weise zu strukturieren oder mit gitterförmigen Elementen zu ummanteln (AT-P 383268); wodurch eine formschlüssige Verankerung mit dem Knochengewebe ermöglicht werden soll. Besonders feste Implantat-Knochen-Verbunde sollen entstehen, wenn unregelmäßige offenzellige spongiosaähnliche Implantate oder Implantatoberflächen Anwendung finden, die eine trabekulären Einwuchs oder Durchwuchs von Knochen in das Implantat ermöglichen.

Nach Henßge (Focus MHL 2(1985)4, 1-8) sind Metallspongiosa Gußkörper aus Kobaltbasislegierungen, die als Solitärimplantate zur Stabilisierung, zu Fusionszwecken bzw. zur Oberflächenstrukturierung für Gelenkersatzimplantate entwickelt wurden. Es ist bekannt, daß bei der Verwendung von Kobaltbasislegierungen kein bindegewebsloser Kontakt mit den Knochen erreicht wird und es zu Abstoßungsreaktionen kommen kann. Außerdem ist die Herstellung der Metallspongiosa-Gußkörper technisch aufwendig und die Oberflächenreinheit ungenügend.

Es sind eine Reihe von Verfahren bekannt, poröse oder schwammartige metallische Körper zu erzeugen. Hierzu werden entweder flüssige heterogene Systeme geschaffen, wie beispielsweise Gemisch aus Gasen oder Salzen und der Metallschmelze, die man erstarren läßt und anschließend die nichtmetallische Phase wieder entfernt. Eine Übersicht über die bekannten Methoden gibt Fritsch, G. (Physik in unserer Zeit 15(1984)5).

So werden in den CH-P 660122 und CH-P 658987 beispielsweise Verfahren zur Herstellung von Knochenersatz beschrieben, die ausgehend von natürlichen oder künstlichen offenzelligen Modellen über aufwendige Arbeitsschritte ein Kernmodell herstellen, dessen Hohlräume schließlich durch Gießen oder Schleudern mit Metall gefüllt werden und anschließend das Kernmaterial wieder entfernt wird. Diese Verfahrensweise hat neben dem großen Aufwand bei der Kernmodellherstellung und den vielen Verfahrensschritten vor allem den Nachteil, daß bei der Herstellung großvolumiger Implantate die Vollständigkeit der Entfernung des Kernmaterials nicht gewährleistet oder schlecht kontrolliert werden kann und daß bei Titanium oder Tantal bzw. deren Legierungen wegen der hohen Schmelzpunkte und der Reaktivität des Flüssigmetalls mit den Formmaterialien zusätzliche Komplikationen auftreten.

Der Erfindung liegt die Aufgabe zugrunde, eine hochfeste schwermetallfreie oder schwermetallarme Metallspongiosa, die über die gesamte Oberfläche mikrostrukturiert und bioaktiv ist und ein bindegewebsloses Einwachsen bzw. Durchwachsen des Knochengewebes fördert und ein Verfahren zur Herstellung von Metallspongiosa unter Ausschluß von Kernformmaterial zu schaffen.

Erfindungsgemäß wird die Aufgabe dadurch gelöst, daß die Metallspongiosa in der erforderlichen Gestalt aus miteinander in regelmäßigen Abständen punktförmig und stoffschlüssig verbundenen, sowie unregelmäßig formschlüssig miteinander verschlauften, hochgradig kaltgeformten Drähten mit quadratischem oder rechteckigem Querschnitt aus Titanium oder Titaniumlegierungen, Tantal oder Tantallegierungen besteht, so daß ein Stützgerüst der Dichte von 50 bis 90 % der Dichte des Substratmaterials mit unregelmäßig strukturierten Zellen vorliegt. Die Zellen haben miteinder Verbindung, so daß der gesamte Implantatkörper einen unregelmäßig durchbrochenen Hohlraum besitzt, der allseitig durch unregelmäßig geformte Kanäle dem einwachsenden Knochengewebe zugängig ist. Die Drähte haben einen Querschnitt bis 3 mm². An Stelle von Drähten aus Reinmetall können auch biokompatible Legierungen, bsw. Tl6Al4V, Verwendung finden.

Die gesamte innere und äußere Metallspongiosaoberfläche ist mit einer bis zu 10 µm starken bioaktiven Schicht versehen, die metallseitig aus reinem Oxid des Substratmaterials besteht und in Richtung zum Hohlraum bzw. zur Umgebung ansteigende Mengen Kalziumphosphat bis zu einer Oberflächenkonzentration von 12 % enthält. Die bioaktive Schicht ist außerdem derart mikrostrukturiert, daß sich pro Quadratmillimeter mindestens 10⁴ Poren mit einem Porendurchmesser > 1 µm und einer maximalen Porentiefe von 2/3 der Bioaktivischichtstärke vorhanden sind.

Die nach außen führenden Kanäle oder der gesamte Hohlraum der Metallspongiosa können zusätzlich vollständig oder teilweise mit resorbierbaren Biomaterialien ausgefüllt sein.

Die Aufgabe der Herstellung wird dadurch gelöst, daß ein rhombenförmig perforiertes kaltverstrecktes Blech, vorzugsweise aus Titanium, Tantal oder deren Legierungen, bsw. in der Form eines an sich bekannten Streckmetalldrahtgitters in dichter Packung entsprechend der gewünschten äußeren Form des Implantates gerollt, gewickelt und/oder gefaltet wird und diese Packung mittels eines Formwerkzeuges in einer Presse zuerst zu > 30 % in Richtung der Hauptausdehnung der Rhomben gestaucht wird, wonach in weiteren Kaltumformvorgängen die endgültige äußere Implantatformgebung erfolgt.

Ein weiteres Merkmal der Erfindung besteht darin, daß die Dichte der Metallspongiosa, die 50 % bis 90 % der Dichte des Titaniums oder der Titaniumlegierung bzw. des Tantals oder der Tantallegierung besitzt, nicht nur durch die Preß-Formgebung, sondern auch durch Einsatz eines unterschiedlich perforierten Bleches und/oder einer differenzierten Lagendichte erreicht wird. Das perforierte Blech wird vor dem Umformen bioaktiv oder bioinert beschichtet.

Die Vorteile der erfindungsgemäßen Lösung bestehen darin, daß eine Metallspongiosa mit quasiisotroper Metalldrahtgitterstruktur entsteht, die sich gegenüber gegossenen Metallgittern aus Titanium bzw. Tantal durch höhere Festigkeit und stark verminderte Kriechneigung auszeichnet.

Werden differenzierte Lagendichten oder unterschiedliche Perforierungen eingesetzt, so entstehen entsprechend differenzierte Dichteverteilungen in der Metallspongiosa, die beispielsweise zu randstabilisierten Implantatformkörpern führen und dem natürlichen Knochen sehr ähnlich sind. Außerdem ist es nicht nur möglich, vor dem formgebenden Pressen bioaktive oder bioinerte Schichten auf dem Metallsubstrat zu erzeugen, sondern auch resorbierbare Biomaterialien in loser Form mit beizugeben, die durch das formgebende Pressen formschlüssig mit verankert werden und je nach Bedarf nach dem Pressen vollständig oder teilweise wieder entfernt werden können.

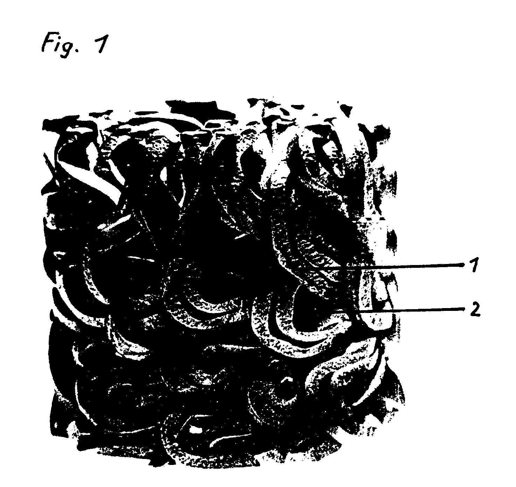

Im folgenden wird die Erfindung an zwei Ausführungsbeispielen erläutert. Figur 1 stellt die Struktur einer Metallspongiosa dar.

### Ausführungsbeispiel 1

Ein rotationssymmetrisch geformter Metallspongiosa-Implantatkörper für eine Defektüberbrückung an einem Röhrenknochen hat eine Gesamtlänge von 42 mm und gliedert sich in drei zylindrische Abschnitte, deren mittlerer 30 mm lange Abschnitt einen Durchmesser von 18 mm aufweist, während die beiden 6 mm langen Enden einen Durchmesser von je 6 mm besitzen. Der Implantatkörper besteht aus einem Stützgerüst von Drähten 1 aus Reinsttitanium mit rechteckigem Querschnitt von 0,5 mm mal 1 mm, welche jeweils im Abstand von 2 mm alternierend mit zwei anderen Drähten 1 stoffschlüssig über eine Länge von 1 mm verbunden sind, so daß zwei benachbarte Drähte 1 jeweils alle 5 mm verbunden sind und keine stoffschlüssig ungebundenen Drähte 1 auftreten.

Die jeweils 2 mm langen nicht stoffschlüssig verbundenen Drahtabschnitte sind in unregelmäßiger Weise umgeformt und miteinander verschlauft, so daß eine offenzellige Drahtgitterstruktur mit unregelmäßig geformtem Hohlraum vorliegt, welcher durch Kanäle 2 unterschiedlicher Form und Größe zugängig ist. Die Dichte des Implantatkörpers, dessen Form durch mehrere Kaltumformungen erreicht wird, beträgt 56 % der Dichte des Reintitaniums. Die gesamte innere und äußere Oberfläche des Implantatkörpers trägt eine 5 µm dicke Titaniumoxidschicht, die an der Oberfläche 7 % Kalziumphosphat enthält. Rasterelektronenmikroskopische Untersuchungen der Oberfläche zeigen auf einer Fläche von 1 mm² 2·10⁴ Poren mit einem Mindestdurchmesser von 1 µm. Die Druckfestigkeit des Implantatkörpers beträgt > 300 N/mm².

### Ausführungsbeispiel 2

Zur Herstellung eines Metallspongiosaimplantatkörpers zur Defektüberbrückung an einem Röhrenknochen wird ein rhombenförmig perforiertes Blech in Form eines Streckmetalls aus EMO-Titan 110 mit folgenden Abmessungen: Breite 25 mm (gemessen in Richtung der Hauptausdehnung der Rhomben), Länge 46 mm (gemessen in Richtung der kurzen Diagonale der Rhomben), einer Maschenlänge von 6 mm (Abstand von Mitte Knotenpunkt zu Mitte Knotenpunkt in Richtung der langen Diagonale), einer Maschenbreite von 4 mm (Abstand von Mitte Knotenpunkt zu Mitte Knotenpunkt in Richtung der kurzen Diagonale), einer Stegbreite von 1 mm sowie einer Stegdicke von 0,5 mm in einem an Kalziumdihydrogenphosphat übersättigten wäßrigen Elektrolyten mit Hilfe der anodischen Oxidation unter Funkenentladung, bei ansteigender Spannung bis 250 V, beschichtet.
Der sorgfältig mit destilliertem Wasser gespülte und getrocknete Zuschnitt wird anschließend zu einem 8 mm dicken und 25 mm langen Zylinder gerollt und in einem mit Titaniumnitrid beschichteten Formwerkzeug in Richtung der Hauptausdehnung der Rhomben zu einem 10 mm langen Metallspongiosaimplantatkörper mit 8 mm Durchmesser gestaucht. Im Anschluß daran wird der Implantatkörper nochmals im gleichen Elektrolyten nachbeschichtet. Die Dichte der so hergestellten Metallspongiosa beträgt 2,6 g/cm³, das entspricht einem Hohlraum von 42 %. Die Druckfestigkeit beträgt > 300 N/mm².

## Patentansprüche

1. Metallspongiosa mit offenzelliger Struktur aus hochschmelzenden Metallen dadurch gekennzeichnet daß die Metallspongiosa in der erforderlichen Gestalt aus miteinander in regelmäßigen Abständen punktförmig und stoffschlüssig verbunden sowie unregelmäßig formschlüssig miteinander verschlauften hochgradig kaltverformten Drähten (1) aus Titanium oder Titaniumlegierungen, Tantal oder Tantallegierungen, mit quadratischem oder rechteckigem Querschnitt besteht, deren Oberfläche allseitig mit einer bis zu 10 µm starken bioaktiven Schicht versehen ist, die metallseitig aus reinem Oxid besteht und in Richtung zur Umgebung ansteigende Mengen an Kalziumphosphat bis zu einer Oberflächenkonzentration von 12 % enthält und in der Weise mikrostrukturiert ist, daß pro Quadratmillimeter mindestens 10⁴ Poren mit einem Durchmesser > 1 µm und einer maximalen Porentiefe von 2/3 der Bioaktivschichtstärke vorhanden sind.

2. Metallspongiosa nach Patentanspruch 1 dadurch gekennzeichnet, daß die nach außen führenden Kanäle (2) oder der gesamte Hohlraum vollständig oder teilweise mit resorbierbaren Biomaterialien ausgefüllt sind.

3. Verfahren zur Herstellung von Metallspongiosa mit offenzelliger Struktur aus hochschmelzenden Metallen dadurch gekennzeichnet, daß ein rhombenförmig perforiertes kaltverstrecktes Blech, vorzugsweise aus Titanium, Tantal oder deren Legierungen in dichter Packung entsprechend der gewünschten äußeren Form des Implantates gerollt, gewickelt und locker gefaltet wird und diese Packung mittels eines Formwerkzeuges zuerst zu > 30 % in Richtung der Hauptausdehnung der Rhomben gestaucht wird und die endgültige äußere Implantatformgebung durch weitere Kaltumformvorgänge erfolgt.

4. Verfahren nach Patentanspruch 3 dadurch gekennzeichnet, daß die Dichte der Metallspongiosa, die 50 % bis 90 % der Dichte des Titaniums oder der Titaniumlegierung bzw. Tantal oder der Tantallegierung besitzt, durch Einsatz eines unterschiedlich perforierten Bleches und/oder einer differenzierten Lagendichte erreicht wird.

5. Verfahren nach den Patentansprüchen 3 und 4 dadurch gekennzeichnet, daß das perforierte Blech vor dem Umformen bioaktiv oder bioinert beschichtet wird.

## Claims

1. Metal spongiosa with open-cell structure made from high-melting metals, characterised in that the metal spongiosa in the required shape consists of high-quality cold-formed wires (1) which are connected to one another at regular intervals at points and in a manner determined by the material, looped together irregularly and positively and made from titanium or titanium alloys, tantalum or tantalum alloys, having square or rectangular cross-section, the surface of which is provided all over with a bioactive layer up to 10 µm thick, which consists of pure oxide with regard to the metal and contains increasing amounts of calcium phosphate up to a surface concentration of 12 % in the direction of the surroundings, and has a microstructure such that at least 10⁴ pores having a diameter > 1 µm and a maximum pore depth of 2/3 of the bioactive layer thickness are present per square millimetre.

2. Metal spongiosa according to patent claim 1, characterised in that the channels (2) leading to the outside or the entire cavity are completely or partially filled with resorbable biomaterials.

3. Process for producing metal spongiosa with open-cell structure made from high-melting metals, characterised in that a rhombus-shaped, perforated cold-formed sheet, preferably of titanium, tantalum or alloys thereof, is rolled, wound and loosely folded in a tight package corresponding to the required external shape of the implant, and this package is compressed by means of a shaping mould initially by 30 % in the direction of the main dimension of the rhombus, and the final external implant shaping is carried out by means of further cold-forming processes.

4. Process according to patent claim 3, characterised in that the density of the metal spongiosa, which has 50 % to 90 % of the density of the titanium or the titanium alloy or tantalum or the tantalum alloy, is achieved by using a sheet with different perforations and/or a different layer density.

5. Process according to patent claims 3 and 4, characterised in that the perforated sheet is bioactively or bioinertly coated before shaping.

## Revendications

1. Structure alvéolaire métallique à cellules ouvertes, en métaux à point de fusion élevé, caractérisée en ce que la structure alvéolaire métallique est composée, sous la forme nécessaire, de fils métalliques (1), reliés ensemble par ponts et avec liaison de la matière, à espacements réguliers, ainsi qu'ayant subi une forte déformation à froid et bouclés ensemble de façon irrégulière, en titane ou en alliages de titane, en tantale ou alliages de tantale, à section transversale carrée ou rectangulaire, dont la surface est pourvue, de tous côtés, d'une couche bioactive d'une épaisseur allant jusqu'à 10 µm, composée, côté métal, d'oxyde pur et contenant, en direction de l'ambiance, des quantités croissantes de phosphate de calcium, en concentration superficielle allant jusqu'à 12 % et dotée d'une microstructure telle que par millimètre carré il y ait au moins 10⁴ pores avec un diamètre > 1 µm et une profondeur de pore maximale de 2/3 de l'épaisseur de la couche bioactive.

2. Structure alvéolaire métallique selon la revendication 1, caractérisée en ce que les canaux (2) menant à l'extérieur ou la totalité du volume creux sont remplis, en totalité ou en partie, de biomatériaux, susceptibles d'être résorbés.

3. Procédé de fabrication de structure alvéolaire métallique à cellules ouvertes, en métaux à point de fusion élevé, caractérisée en ce qu'une tôle perforée étirée à froid et de forme rhomboïdale, de préférence en titane, tantale ou leurs alliages, est laminé pour former une garniture compacte, en fonction de la forme extérieure souhaitée de l'implant, puis enroulée et pliée de façon plus lâche, cette garniture étant d'abord comprimée au moyen d'un outil de formage, pour être réduite à 30 % en direction de la grande dimension des losanges et la configuration extérieure finale de l'implant étant obtenue au moyen d'autres processus de formage à froid.

4. Procédé selon la revendication 3, caractérisé en ce que la densité de la structure alvéolaire métallique, qui est de 50 % à 90 % de la densité du titane ou des alliages de titane, respectivement du tantale ou des alliages de tantale, est atteinte par mise en oeuvre d'une tôle à perforation différente et/ou une densité de couche différente.

5. Procédé selon les revendications 3 et 4, caractérisé en ce que la tôle perforée est revêtue, avant façonnage, d'un revêtement bioactif ou bioinerte.
